# EUROPÄISCHE PATENTANMELDUNG

(11) **EP 1 028 104 A1**
(43) Veröffentlichungstag der Anmeldung: **16.08.2000**
(21) Anmeldenummer: 00101607.0
(22) Anmeldetag: 27.01.2000
(51) Int. Cl.: C07C 209/48, C07C 209/60, C07C 211/13

(54) **Verfahren zur Herstellung von 2-Aminomethyl-1,5-pentandiamin**

(30) Priorität: 09.02.1999 DE 19905277
(71) Anmelder: BAYER AG, 51368 Leverkusen (DE)
(72) Erfinder: Fischer, Konrad, Dr., 51519 Odenthal (DE); Richter, Frank, Dr., 51373 Leverkusen (DE); Bazanov, Anatoly, Prof., 193318 St. Petersburg (RU); Timofeev, Alexandre, Dr., 195253 St. Petersburg (RU); Zubritskaya, Natalja, Dr., 190068 St. Petersburg (RU); Smirnova, Galina, Dr., 192283 St. Petersburg (RU)

(57) **Zusammenfassung**

Die Erfindung betrifft ein Verfahren zur Herstellung von 2-Aminomethyl-1,5-pentandiamin durch simultane Umsetzung von 2,4-Dicyano-1-buten mit Ammoniak und Wasserstoff ohne Isolierung von Zwischenprodukten. Das 2-Aminomethyl-1,5-pentandiamin kann als Zwischenprodukt bei der Herstellung von Lacken, Tensiden, Waschmitteln und als Bestandteil von Epoxidzusammensetzungen eingesetzt werden.

## Beschreibung

Die Erfindung betrifft ein Verfahren zur Herstellung von 2-Aminomethyl-1,5-pentandiamin. Dieses kann als Zwischenprodukt bei der Herstellung von Lacken, Tensiden, Waschmitteln und als Bestandteil von Epoxidzusammensetzungen eingesetzt werden.

Die Herstellung von 2-Aminomethyl-1,5-pentandiamin durch Hydrierung von 2-Aminomethyl-1,5-pentandinitril im ammoniakalischen Medium wird in *Jap. Pat. 46-2484 (1971)* beschrieben. Der Hauptnachteil dieses Verfahrens ist, daß es von der Verwendung von 2-Aminomethyl-1,5-pentandinitril als Ausgangsstoff ausgeht, das durch ein kompliziertes Synthese- und Isolierverfahren hergestellt werden muß. Hinzu kommt, daß 2-Aminomethyl- 1,5-pentandinitril, zugänglich beispielsweise durch Addition von Ammoniak an 2,4-Dicyano-1-buten (dimeres Acrylnitril), einen sehr hohen Siedepunkt besitzt und nur begrenzt stabil ist. Seine Isolierung und Handhabung sind dadurch extrem erschwert, Produktverluste und ein hoher Energieverbrauch sind bei seiner Herstellung und Verarbeitung unvermeidbar.

Ziel der Erfindung war die Entwicklung eines wirtschaftlicheren Verfahrens zur technischen Herstellung von 2-Aminomethyl-1,5-pentandiamin durch die katalytische Reaktion von 2,4-Dicyano-1-buten mit Ammoniak und Wasserstoff in einer Einstufenreaktion ohne Isolierung des ggf. gebildeten Zwischenproduktes 2-Aminomethyl-1,5-pentandinitril nach dem folgenden Schema:

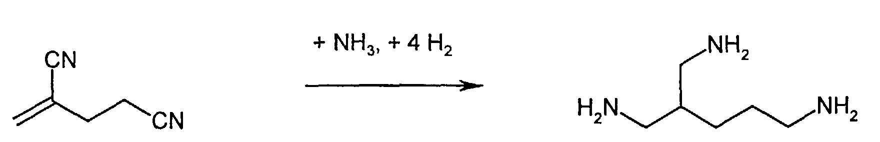

Gegenstand der Erfindung ist ein Verfahren zur Herstellung von 2-Aminomethyl-1,5-pentandiamin, dadurch gekennzeichnet, daß die Umsetzung, ausgehend von 2,4-Di-cyano-1-buten, Wasserstoff und Ammoniak, in einem Schritt erfolgt.

Gegenstand der Erfindung ist ferner des Verfahren zur Herstellung von 2-Amino-methyl-1,5-pentandiamin durch Umsetzung von 2,4-Dicyano-1-buten, Wasserstoff und Ammoniak in einem, mit dem Hydrierkatalysator gefüllten Festbettrohrreaktor, wobei die Edukte vorher in einem Durchflußbehälter gemischt werden und kobalt- und/oder nickelhaltige Kontakte als Hydrierkatalysatoren eingesetzt werden.

Das 2,4-Dicyano-1-buten kann dabei mit oder ohne die Verwendung von Lösungsmitteln umgesetzt werden. Als Lösungsmittel eignen sich hierfür beispielsweise aliphatische Alkohole mit 1 bis 6 Kohlenstoffatomen, bevorzugt 1 bis 4 Kohlenstoffatomen.

Die Umsetzung kann im Temperaturbereich von 20 bis 200 °C geführt werden, bevorzugt wird zwischen 40 und 160°C, besonders bevorzugt zwischen 50 und 100°C gearbeitet.

Der Wasserstoffdruck kann 50 bis 250 atm betragen, bevorzugt wird zwischen 100 und 220 atm hydriert.

Das erfindungsgemäße Verfahren zur Herstellung von 2-Aminomethyl-1,5-pentandiamin aus 2,4-Dicyano-1-buten, Ammoniak und Wasserstoff hat gegenüber den bekannten Verfahren des Standes der Technik den Vorteil, daß es in einem einzigen Schritt, ausgehend von wohlfeilen Edukten und ohne die aufwendige Isolierung und Reinigung von Zwischenprodukten durchgeführt werden kann.

Gegebenenfalls kann der katalytische Festbettreaktor mit feststehendem Hydrierkatalysator kontinuierlich betrieben werden, wobei die Reaktionskomponenten zuvor in einem Durchflußbehälter kontinuierlich gemischt werden. Das aus dem Reaktor austretende Reaktionsgemisch wird auf Umgebungstemperatur abgekühlt, das überschüssige Wasserstoff-Ammoniak-Gasgemisch abgetrennt und in das Verfahren zurückgeführt, anschließend werden aus den flüssigen Reaktionsprodukten Ammoniak sowie ggf. verwendete Lösungsmittel abdestilliert und der Rückstand rektifiziert. Hierbei werden im wesentlichen erhalten: nicht umgesetztes 2,4-Dicyano-1-buten, 3-Aminomethyl-piperidin und 2-Methyl-1,5-pentandiamin. Das auf diese Weise hergestellte 2-Aminomethyl-1,5-pentandiamin enthält nach gaschromatografischer Analyse mindestens 95 Gew.-%, bevorzugt über 98 Gew.-% der Hauptverbindung.

Zur Gewährleistung einer hohen Standzeit des Katalysators bei gleichbleibend hoher Aktivität und insbesondere Selektivität zur Bildung von 2-Aminomethyl-1,5-pentandiamin werden bevorzugt Alkalimetalloxid-dotierte Kobaltkatalysatoren verwendet. Hierbei werden besonders bevorzugt Kobaltkatalysatoren verwendet, die mit einem Alkalimetalloxid als Oberflächen-Additiv dotiert worden sind, wobei die Menge an Additiv im Bereich von 1-15 Gew.-%, bevorzugt 1-10 Gew.-%, bezogen auf die Gesamtmenge an Katalysator, liegt.

### Beispiele

Alle %-Angaben beziehen sich auf die Masse.

### Beispiel, 1

Ein Rohrreaktor wurde mit 200 cm³ eines granulierten Katalysators auf Kobaltbasis, der 65 % Co, 3,5 % Mn und 3 % H₃PO₄ enthielt, beladen, und der Katalysator wurde reduziert. Danach wurden 10 ml einer 50 %igen Lösung 2,4-Dicyano-1-buten in 2-Propanol und 60 ml flüssigen Ammoniaks bei 25°C in den unteren Teil eines Mischrohres mit einem Volumen von 500 cm³ eingeführt und Wasserstoff mit einer Geschwindigkeit von 100 l/h eingeleitet. Das entstehende Gas-Flüssigkeitsgemisch wurde in den Kopf des Reaktors bei 80°C und 200 atm eingeleitet. Das aus dem unteren Teil des Reaktors abgeführte Reaktionsgemisch wurde abgekühlt, das zurückgeführte Wasserstoff-Ammoniak-Gasgemisch abgetrennt und das Ammoniak aus den flüssigen Produkten abgezogen. Anschließend wurde 2-Propanol im Vakuum abdestilliert und der Rückstand zur Abtrennung von Verunreinigungen und nicht umgesetztem Edukt (2-Methyl-1,5-pentandiamin, 3-Aminomethylpiperidin und 2,4-Dicyano-1-buten) aus dem Rohtriamin rektifiziert. Das Produkt wurde als Fraktion mit einem Siedepunkt von 115 bis 117°C (3-4 mm) aufgefangen. Die Ausbeute an 2-Aminomethyl-1,5-pentandiamin betrug in diesem Versuch 63 %, bezogen auf das umgesetzte 2,4-Dicyano-1-buten.

### Beispiel 2

Zu einem Katalysator wie unter 1. beschrieben werden 10 % Natriumoxid als Oberflächen-modifizierendes Additiv gegeben. 200 cm³ dieses Katalysators werden in den Reaktor gegeben und das in Beispiel 1 beschriebene Verfahren wird wiederholt. Es werden 1,9 kg 2-Aminomethyl-1,5-pentandiamin mit einer Reinheit von 98 % erhalten. Die Ausbeute, bezogen auf eingesetztes 2,4-Dicyano-1-buten, beläuft sich auf 66,6 %.

## Patentansprüche

1. Verfahren zur Herstellung von 2-Aminomethyl-1,5-pentandiamin durch simultane Umsetzung von 2,4-Dicyano-1-buten mit Ammoniak und Wasserstoff, dadurch gekennzeichnet, daß diese Umsetzung in einem Schritt ohne die Isolierung von Zwischenprodukten erfolgt.

2. Verfahren nach Anspruch 1, dadurch gekennzeichnet, daß die Reaktion in einem, mit einem Hydrierkatalysator gefüllten, katalytischen Festbettrohrreaktor durchgeführt wird, wobei die Edukte vorher in einem Durchflußbehälter gemischt werden, kobalt- und/oder nickelhaltige Kontakte als Hydrierkatalysatoren eingesetzt werden und die Umsetzung im Temperaturbereich zwischen 20 und 200°C bei einem Druck zwischen 50 und 250 atm durchgeführt wird.

3. Verfahren nach Anspruch 2, dadurch gekennzeichnet, daß die Umsetzung im Temperaturbereich von 50 bis 100 °C geführt wird.

4. Verfahren nach Anspruch 2, dadurch gekennzeichnet, daß der Druck zwischen 100 und 220 atm liegt.

5. Verfahren nach Anspruch 1 bis 4, dadurch gekennzeichnet, das als Katalysator kobalt- und/oder nickelhaltige Kontakte zum Einsatz kommen, die mit 1-15 Gew.-% eines Alkali- und/oder Erdalkalimetalloxides dotiert sind.

6. Verfahren nach Anspruch 5, dadurch gekennzeichnet, daß als Dotierungsmittel Natriumoxid verwendet wird.
